# EUROPEAN PATENT SPECIFICATION

(11) **EP 3 881 856 B1**
(45) Date of publication and mention of the grant of the patent: **07.08.2024**
(21) Application number: 19875343.6
(22) Date of filing: 25.10.2019
(51) Int. Cl.: A61K 38/19, A61K 38/16, A61K 38/36, A61P 7/06

(54) **PHARMACEUTICAL COMPOSITION FOR TREATING APLASTIC ANEMIA**
PHARMAZEUTISCHE ZUSAMMENSETZUNG ZUR BEHANDLUNG VON APLASTISCHER ANÄMIE
COMPOSITION PHARMACEUTIQUE DESTINÉE AU TRAITEMENT DE L'ANÉMIE APLASIQUE

(30) Priority: 26.10.2018 JP 2018202097
(43) Date of publication of application: 22.09.2021
(73) Proprietor: Kyowa Kirin Co., Ltd., Tokyo 100-0004 (JP)
(72) Inventor: TSUJI Yukie, Tokyo 100-0004 (JP); KODAMA Miyako, Tokyo 100-0004 (JP)
(74) Representative: Hoffmann Eitle
(86) International application number: PCT/JP2019/041814
(87) International publication number: WO 2020/085467

(56) References cited:
- WO-A1-02/15926
- JP-A- 2002 536 960
- KIRIN KYOWA ET AL: "Study Details Tabular View No Results Posted Disclaimer How to Read a Study Record Trial record 1 of 7 for: romiplostim | Aplastic Anemia Previous Study | Return to List | Next Study Study of Romiplostim(AMG531) in Subjects With Aplastic Anemia Sponsor: Study Description Brief Summary", 1 January 2016 (2016-01-01), XP055922686, Retrieved from the Internet <URL:https://clinicaltrials.gov/ct2/show/NCT02773290> [retrieved on 20220518]
- YOUNG NEAL S. ET AL: "Aplastic Anemia", THE NEW ENGLAND JOURNAL OF MEDICINE, vol. 379, no. 17, 25 October 2018 (2018-10-25), US, pages 1643 - 1656, XP055922675, ISSN: 0028-4793, Retrieved from the Internet <URL:https://www.ncbi.nlm.nih.gov/pmc/articles/PMC6467577/pdf/nihms-995041.pdf> DOI: 10.1056/NEJMra1413485
- TOMIYAMA YOSHIAKI ET AL: "Efficacy and Safety of Romiplostim in Patients with Acquired Aplastic Anemia Ineligible or Refractory to Immunosuppressive Therapy: Interim Analysis of Phase 2/3 Clinical Trial", BLOOD, AMERICAN SOCIETY OF HEMATOLOGY, US, vol. 132, 29 November 2018 (2018-11-29), pages 1306, XP086592553, ISSN: 0006-4971, DOI: 10.1182/BLOOD-2018-99-112478
- JANG JUN HO ET AL: "Efficacy and safety of romiplostim in refractory aplastic anaemia: a Phase II/III, multicentre, open-label study", BRITISH JOURNAL OF HAEMATOLOGY, vol. 192, no. 1, 5 November 2020 (2020-11-05), Hoboken, USA, pages 190 - 199, XP093027756, ISSN: 0007-1048, Retrieved from the Internet <URL:https://onlinelibrary.wiley.com/doi/full-xml/10.1111/bjh.17190> [retrieved on 20230310], DOI: 10.1111/bjh.17190
- JONG WOOK LEE , JUN HO JANG , SUNG-EUN LEE , CHUL WON JUNG , SILVIA PARK , IL-HOAN OH: "Efficacy and Safety of Romiplostim in Patients with Aplastic Anemia Refractory to Immunosuppressive Therapy: 1-Year Interim Analysis of Phase 2 Clinical Trial", BLOOD, vol. 128, no. 22, 3910, 2 December 2016 (2016-12-02), pages 1 - 2, XP009527617, ISSN: 0006-4971, DOI: 10.1182/blood.V128.22.3910.3910
- JONG-WOOK LEE , SUNG-EUN LEE , CHUL WON JUNG , SILVIA PARK , JIN-A KIM , IL-HOAN OH, JUN HO JANG: "Hematologic Response to Romiplostim Treatment Is Associated with Stimulation of Primitive Stem/Progenitor Cells and Stromal Cells in Patients with Aplastic Anemia Refractory to Immunosuppressive Therapy: A 2-Year Interim Exploratory Analysis of a Phase 2 Clinical Trial", BLOOD, vol. 130, no. 1, 1167, 7 December 2017 (2017-12-07), pages 1 - 3, XP009527620, DOI: 10.1182/blood.V130.Suppl_1.1167.1167
- ARIA SHUNYA : "Reference Guide for Aplastic Anemia Practice", 30 May 2018 (2018-05-30), pages 1 - 28, XP055806054, Retrieved from the Internet <URL:http://zoketsushogaihan.com/file/guideline_H30/02.pdf> [retrieved on 20191127]

## Description

### Technical Field

### [Related Application]

The present description refers to the contents as disclosed in the description of Japanese Patent Application No. 2018-202097 (filed on October 26, 2018), which is a priority document of the present application.

### [Technical Field]

The present invention relates to Romiplostim for treating aplastic anemia as claimed

### Background Art

Aplastic anemia (AA) is a disease characterized by a reduction in the number of all blood cells in the peripheral blood (pancytopenia) and a decrease in bone marrow cell density (hypoplasia). Subjective symptoms of AA include anemia symptoms such as shortness of breath during effort, palpitations and dizziness, fever due to infection, and bleeding tendency such as subcutaneous ecchymoses, ulorrhagia and epistaxis. Objective findings include facial pallor, anemia-like palpebral conjunctiva, subcutaneous hemorrhage and ulorrhagia. AA can be fatal due to the transition to myelodysplastic syndrome (MDS) or acute myeloid leukemia (AML), to a bleeding tendency or to an infection. In Japan, it was designated as a designated intractable disease by the Ministry of Health, Labor and Welfare in 1972.

The treatment of AA includes supportive therapy such as transfusion of platelets and red blood cells, administration of hematopoietic factors such as granulocyte colony-stimulating factor (G-CSF), and immunosuppressive therapy, anabolic steroid therapy, hematopoietic stem cell transplantation as treatments aiming at the restoration of hematopoietic function. Since the prognosis and treatment policy differ greatly depending on the severity of AA, the treatment guidelines are indicated according to the severity.

In the treatment guidelines in Japan, it is recommended to start the treatment with cyclosporin (Cyclosporin A; CsA) for AA patients with stages 1 to 2a (mild to moderate with no transfusion required) (except for patients with a platelet count of 100000/µL or more and having only anemia and a decreased neutrophil count), and to examine its effects. The treatment response with cyclosporin appears within 8 weeks at the latest. If no increase in the platelet count or reticulocyte count is observed within 8 weeks after treatment, appropriate treatment such as the combined use of anti-human thymocyte immunoglobulin (ATG) or Eltrombopag (EPAG) will be selected depending on the progress of cytopenia, the need for transfusion, the presence or absence of subjective symptoms, etc..

For AA patients with stages 2b to 5 (moderate requiring transfusion to most severe), hematopoietic stem cell transplantation is the main treatment for patients having an HLA-matched sibling donor who are younger than 40 years. For patients for whom transplantation is not indicated and patients aged 40 years or older, treatment by immunosuppressive therapy such as ATG and CsA is mainly performed and the combined use of EPAG is considered as necessary.

However, with hematopoietic stem cell transplantation, transplant-related death appears in 10 to 200 of the patients, and with immunosuppressive therapy, about 5 to 10% of the long-term survivors transition to myelodysplastic syndromes (MDS) or acute myeloid leukemia (AML) despite response to treatment.
The response rate of immunosuppressive therapy is about 33 to 57%. Therefore, for patients who are refractory to immunosuppressive therapy, for whom immunosuppressive therapy is not indicated, supportive therapy centered on transfusion or administration of hematopoietic factors is provided to prolong the life. If the degree of anemia or thrombocytopenia is severe, transfusion is performed if the clinical symptoms associated therewith are moderate or more, but it is necessary to minimize the transfusion to avoid the risk of unknown infections, expression of platelet transfusion refractoriness due to anti-HLA antibody production and rejection during hematopoietic stem cell transplantation. G-CSF is administered in patients with neutrophil counts of 500/µL or less who are at high risk for severe infections, but the effect is temporary.

Although it was expected that the treatment results would improve by the combined use of EPAG with severe AA patients refractory to immunosuppressive therapy, there were patients who did not respond even when using EPAG in Japanese AA patients not treated with ATG in the clinical trial data of EPAG. In addition, in clinical practice, there may also be patients for whom the administration of EPAG is a concern, such as patients with a liver disorder and patients who are old and cannot maintain medication compliance due to orally administered preparations. Thus, there are patients for whom even drug therapy aiming to restore hematopoietic function by EPAG cannot be expected to be effective, and a safe and more effective treatment is needed.

Romiplostim is a platelet hematopoietic stimulating factor agent that binds to c-Mpl, which is a receptor for endogenous thrombopoietin (TPO) to enhance platelet production (Patent Literature 1). After being approved in Australia in July 2008 as a therapeutic drug for "Thrombocytopenia in Adults with Chronic Immune (Idiopathic) Thrombocytopenic Purpura (ITP)", Romiplostim has been approved in more than 60 countries and is manufactured and sold under the trade names such as Romiplate (R).

c-Mpl is expressed not only in megakaryocytic precursor cells but also in more undifferentiated hematopoietic stem/precursor cells in bone marrow, and it has been suggested that TPO promotes the production of multilineage blood cells by activating c-Mpl. Therefore, Romiplostim is expected to be effective on AA, and clinical development has been started targeting at adult AA patients. A phase II clinical (Ph2) trial of Romiplostim targeted at AA patients refractory to immunosuppressive therapy was started in 2014 (Non Patent Literature 1), and a phase II/III clinical (Ph2/3) trial was started in 2016 (Non Patent Literature 2). In these clinical trials, Romiplostim was subcutaneously administered once a week, and its drug efficacy was verified using platelets, erythroid and neutrophil response, weaning from platelet transfusion, etc. as indicators and it was found that an initial dose of 10 µg/kg was the most effective.

### Citation List

### Patent Literature

Patent Literature 1: WO 2000/024770

### [Non Patent Literature]

Non Patent Literature 1: Lee et al., "Efficacy and Safety of Romiplostim in Patients with Aplastic Anemia Refractory to Immunosuppressive Therapy: 1-Year Interim Analysis of Phase 2 Clinical Trial" Blood (2016) 128:3910
Non Patent Literature 2: Lee et al., "Hematologic Response to Romiplostim Treatment Is Associated with Stimulation of Primitive Stem/Progenitor Cells and Stromal Cells in Patients with Aplastic Anemia Refractory to Immunosuppressive Therapy: A 2-Year Interim Exploratory Analysis of a Phase 2 Clinical Trial" Blood (2017) 130:1167

### Summary of Invention

### Technical Problem

An object of the present invention is to provide Romiplostim for use in an effective method for treating aplastic anemia as claimed

### Solution to Problem

As a result of intensive studies, the inventors have found that high efficacy and safety can be obtained in the treatment of aplastic anemia by administering Romiplostim at a specific dose and administration, and completed the present invention.

The present invention is set out in the claims.

### Advantageous Effects of Invention

According to the present invention, high drug efficacy can be achieved in the treatment with Romiplostim in patients with aplastic anemia who do not respond to immunosuppressants.

### Brief Description of Drawings

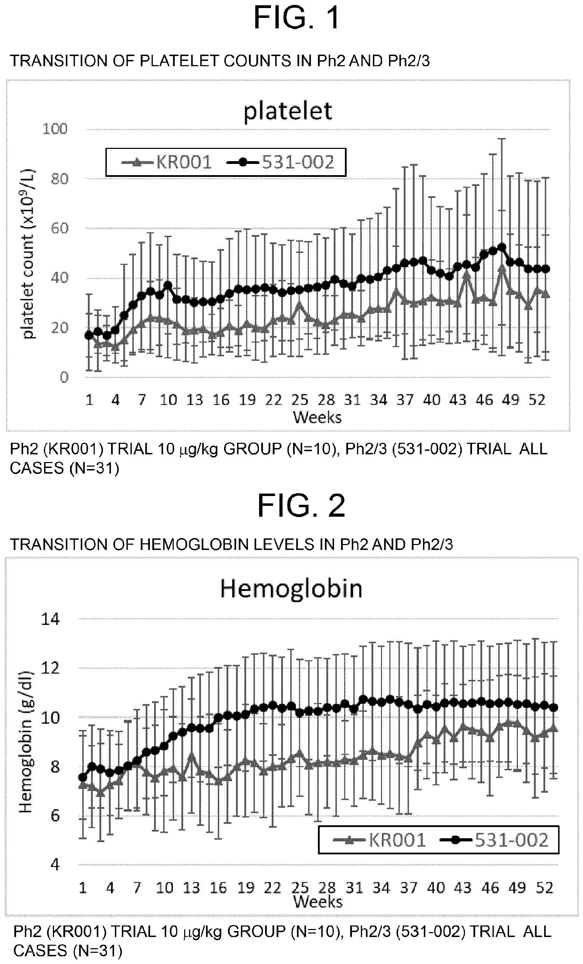

[Figure 1] Figure 1 indicates the transition of platelet counts in Ph2 and Ph2/3. In the graph, the lower light-colored line indicates the average value for the group starting at 10 µg/kg in the Ph2 (KR001) trial (N=10) and the upper dark-colored line indicates the average value for all the cases of the Ph2/3 (531-002) trial (N=31). The error bars indicate the standard deviations. The horizontal axis indicates the number of weeks from the start of administration and the vertical axis indicates the platelet count.
[Figure 2] Figure 2 indicates the transition of hemoglobin levels in Ph2 and Ph2/3. In the graph, the lower light-colored line indicates the average value for the group starting at 10 µg/kg in the Ph2 (KR001) trial (N=10) and the upper dark-colored line indicates the average value for all the cases of the Ph2/3 (531-002) trial (N=31). The error bars indicate the standard deviations. The horizontal axis indicates the number of weeks from the start of administration and the vertical axis indicates the hemoglobin level.

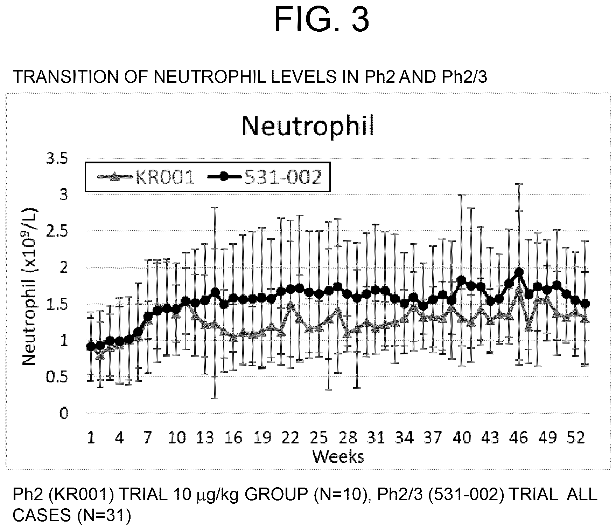

[Figure 3] Figure 3 indicates the transition of neutrophil levels in Ph2 and Ph2/3. In the graph, the lower light-colored line indicates the average value for the group starting at 10 µg/kg in the Ph2 (KR001) trial (N=10) and the upper dark-colored line indicates the average value for all the cases of the Ph2/3 (531-002) trial (N=31). The error bars indicate the standard deviations. The horizontal axis indicates the number of weeks from the start of administration and the vertical axis indicates the neutrophil count.

### Description of Embodiments

### "Romiplostim"

Romiplostim is a genetically-modified fusion protein having a molecular weight of about 59 kDa and is a dimer composed of two subunit molecules consisting of 269 amino acid residues (SEQ ID No. 1). The 2nd to 228th amino acids of the 269 amino acids of the monomer constitute a human IgG1 Fc domain, and the 229th to 269th amino acids constitute a peptide containing a human thrombopoietin receptor (c-Mpl) binding sequence. In Romiplostim, single chains in which a peptide chain containing two c-Mpl binding domains and the C-terminus of the Fc domain of human IgG1 are linked, are linked by a disulfide bond to form a dimer. 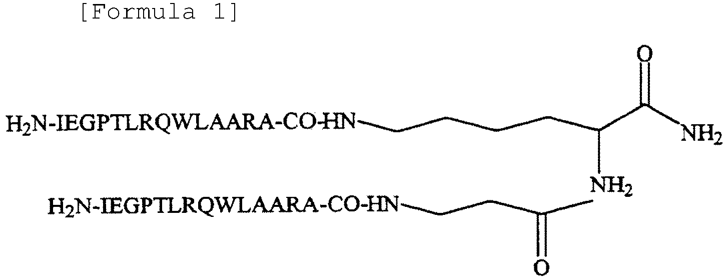
C2634H4086N722O790S18
Mw: 59085

Romiplostim has platelet-producing activity and/or megakaryocyte-producing activity. Romiplostim acts as a platelet hematopoietic stimulating factor preparation by binding to c-Mpl, which is a receptor for endogenous thrombopoietin (TPO) and enhancing platelet production. TPO is a human megakaryocyte/platelet hematopoietic stimulating factor and was cloned in 1994. Romiplostim is thought to promote cell proliferation and differentiation in the process from bone marrow precursor cells to megakaryocytes and as a result to increase the number of platelets (hereinafter also referred to as PLT), by binding to c-Mpl and activating it.

In the present invention, the method for producing Romiplostim is not particularly limited as long as it is pharmaceutically acceptable.

### "Aplastic Anemia"

Aplastic anemia (AA) is a disease characterized by a reduction in the number of all blood cells in the peripheral blood (pancytopenia) and a decrease in bone marrow cell density (hypoplasia). In fact, it is diagnosed as AA by excluding other diseases with clearer concepts, which have similar characteristics. However, the essence of the disease can be said to be "a state in which hematopoietic stem cells are continuously decreasing, even though there is no effect of drugs showing bone marrow toxicity."

To diagnose AA, it is necessary to observe anemia, a bleeding tendency and sometimes fever as clinical findings, and to meet at least two of these three items: hemoglobin (Hb) concentration less than 10 g/dL, neutrophils less than 1500/µL and platelets less than 100000/µL. Furthermore, it is diagnosed as AA if diseases causing cytopenia, bone marrow hypoplasia and other pancytopenias can be ruled out, but differentiation from MDS can be sometimes difficult.

AA is divided into congenital AA and acquired AA according to the cause. The most frequent congenital AA is Fanconi anemia, which is an autosomal recessive hereditary disease and is characterized by skeletal deformities, short stature and malformations such as gonadal dysfunction, in addition to bone marrow hypoplasia. In addition, acquired AA includes idiopathic (primary) forms, secondary forms caused by various drugs and radiation exposure/chemical substances such as benzene, and special forms associated with onset after hepatitis and paroxysmal nocturnal hemoglobinuria. Most of acquired AA are considered to be idiopathic (primary) in Japan.

The treatment of AA includes supportive therapy such as transfusion of platelets and red blood cells, administration of hematopoietic factors such as G-CSF, and immunosuppressive therapy, anabolic steroid therapy and hematopoietic stem cell transplantation as treatments aiming at the restoration of hematopoietic function. Since the prognosis and treatment policy differ greatly depending on the severity of AA, the treatment guidelines are indicated according to the severity. As described above, treatment with CsA is recommended for AA patients with stages 1 to 2a (mild to moderate with no transfusion required), and ATC or EPAG is used in combination as appropriate. For AA patients with stages 2b to 5 (moderate requiring transfusion to most severe), hematopoietic stem cell transplantation or immunosuppressive therapy or a combination of immunosuppressive therapy and EPAG is considered. For patients who are refractory to immunosuppressive therapy, or for whom immunosuppressive therapy is not indicated, supportive therapy centered on transfusion and hematopoietic factors is adopted.

### "Pharmaceutical composition disclosed herein"

The "pharmaceutical composition for treating aplastic anemia, comprising Romiplostim as an active ingredient" disclosed herein (hereinafter referred to as "the pharmaceutical composition disclosed herein") is administered subcutaneously (subcutaneous injection) to AA patients by intravenous administration (also referred to as intravenous injection) or by intravenous drip infusion (also referred to as intravenous drip injection or drip infusion).

The pharmaceutical composition disclosed herein comprises one or more pharmacologically acceptable carriers, additives, pH adjusters and the like. Examples of additives include tonicity agents, buffers, solubilizers and preservatives.

A tonicity agent is not particularly limited, and examples thereof include sodium chloride, calcium chloride, potassium chloride, magnesium chloride, fructose, glucose and D-mannitol.

Examples of buffers include a composition comprising potassium dihydrogen phosphate, dipotassium hydrogen phosphate, trisodium phosphate, sodium dihydrogen phosphate and disodium hydrogen phosphate, sodium citrate and sodium citrate hydrate.

Examples of solubilizers include ethanol, ethylenediamine, capric acid, L-glutamic acid, L-lysine, calcium oxide, magnesium oxide, sorbitan sesquioleate, D-sorbitol, nicotinic acid amide, propylene glycol, polysorbate 80 and Lauromacrogol (9E.O.).

Examples of preservatives include phenol, sodium edetate, benzalkonium chloride, chlorocresol, chlorobutanol, sodium salicylate, ethyl para-hydroxybenzoate and butyl para-hydroxybenzoate.

Examples of pH adjusters include hydrochloric acid, dilute hydrochloric acid, glycine, succinic acid, phosphoric acid, phosphate, acetic acid, tartaric acid and meglumine.

The form of the pharmaceutical composition disclosed herein is not particularly limited, and may be a ready-to use preparation for subcutaneous administration or may be a preparation prepared when needed by diluting or dissolving lyophilized Romiplostim in a solvent for injection such as water.

The pharmaceutical composition disclosed herein is provided by filling in a glass container, a plastic container, and the like. The shape of the container is not particularly limited, and examples thereof include vials, syringes, bags and bottles.

One preferable example of the pharmaceutical composition disclosed herein is a powdery preparation obtained by lyophilizing Romiplostim with pharmacologically acceptable additives according to a conventional method. Specific examples of such pharmaceutical composition include Romiplate (R). Romiplate (R) is a freeze-dried product of Romiplostim containing D-mannitol, refined sucrose, L-histidine, polysorbate 20 and dilute hydrochloric acid as additives and is used by dissolving it as appropriate in water for injection before administration.

### "Dosage and administration of the pharmaceutical composition disclosed herein"

The pharmaceutical composition disclosed herein is subcutaneously administered once a week at a fixed dose of 10 µg/kg/week of Romiplostim for 4 weeks from the start of administration, and is increased or decreased as appropriate according to the condition of the patient and in accordance with the claims from week 5.

In a first embodiment, the pharmaceutical composition disclosed herein is administered so that Romiplostim is administered at 10 µg/kg/week for 4 weeks from the start of administration, and administered at more than 15 µg/kg/week and a maximum of 20 µg/kg/week from week 5.

In a second embodiment, the pharmaceutical composition disclosed herein is administered so that Romiplostim is administered at 10 µg/kg/week for 4 weeks from the start of administration, administered at 15 µg/kg/week from week 5 to week 8, and administered at a maximum of 20 µg/kg/week from week 9, and the same dose is maintained for 4 weeks or more after a dose change.

In a third embodiment, the pharmaceutical composition disclosed herein is administered so that Romiplostim is administered at 10 µg/kg/week for 4 weeks from the start of administration, administered at 15 µg/kg/week from week 5 to week 8, and administered at 20 µg/kg/week from week 9 to week 12, and the same dose is maintained for 4 weeks or more after a dose change.

In a fourth embodiment, the pharmaceutical composition disclosed herein is administered so that Romiplostim is administered at 10 µg/kg/week for 4 weeks from the start of administration, and administered at 15 or 20 µg/kg/week from week 5, and the same dose is maintained for 4 weeks or more after a dose change.

In a fifth embodiment, the pharmaceutical composition disclosed herein is administered so that Romiplostim is administered at 10 µg/kg/week for 4 weeks from the start of administration, and administered with a dose increment of Romiplostim from week 5 being 5 µg/kg/week. Preferably, the same dose is maintained for 4 weeks or more after a dose change.

Preferably, the same dose is maintained for 4 weeks or more after the dose change.

Preferably, the dose increment of Romiplostim from week 5 is 5 µg/kg/week.

For safe administration, platelet counts are measured at the beginning of use of the pharmaceutical composition disclosed herein and at the time of dose adjustment, preferably once a week. Even if the dose is maintained, it is preferable to measure the platelet count roughly once every 4 weeks.

The dose of Romiplostim can be increased or decreased as appropriate within a range not exceeding 5 µg/kg. For example, the dose is increased when no platelet response (when the platelet count has increased by 20,000/µL or more, or when the platelet count is 10,000/µL or more and has increased by 100% or more from the baseline, or when the patients is platelet transfusion independent for 8 consecutive weeks) is observed even if the same dose is continuously administered for 4 weeks, and when it is considered that there is no problem in safety.

For the dose adjustment, it is preferable to use the minimal dose necessary for treatment, while referring to the table below.

**[Table 1]**

| Platelet count | Adjustment method |
|---|---|
| 200,000/µL to 400,000/µL | Decrease the dose |
| More than | Discontinue. After discontinuation, if the platelet count has decreased to 200,000/µL, in principle, the dose is decreased by 5 µg/kg from the dose before discontinuation and the administration is resumed. When the dose before discontinuation is 5 µg/kg or less, if the platelet count has decreased to 50,000/µL, the administration is resumed at the same dose as before discontinuation. |
| 400,000/µL | |

When the improvement of the blood cell lineages (for example, platelet count over 50,000/µL under transfusion independence, hemoglobin concentration over 10 g/dL under transfusion independence, and neutrophil count over 1,000/µL) lasts for 8 weeks or more, it is preferable to reduce the pharmaceutical composition disclosed herein within a range not exceeding 5 µg/kg of Romiplostim. For example, if the improvement of the tri-lineage is maintained for 4 weeks with the reduced dose, the dose of Romiplostim is further decreased within a range not exceeding 5 µg/kg, and the dose reduction is considered every 4 weeks thereafter. In addition, it is preferable to discontinue if the improvement of the tri-lineage is maintained for 4 weeks with a dose of less than 5 µg/kg Romiplostim. If deterioration of any of the 3 blood cells is observed during discontinuation, the treatment can be resumed with the dose before discontinuation. If no platelet response is observed even if a maximum dose of 20 µg/kg/week is administered for 8 consecutive weeks, appropriate measures are taken, such as stopping the administration.

The dose and administration according to the present invention has a short fixed dose period (the dose can be increased early on), a large increase range (the dose can be increased at once), and can reach the maximum dose early on. By administering Romiplostim according to the dose and administration of the present invention, high drug efficacy (increased platelet, hemoglobin level and/or neutrophil level, or tri-lineage response) can be obtained, and the effect continues even after reaching the maximum dose. Therefore, according to the present invention, an excellent improvement effect is achieved even in AA patients who are refractory to immunosuppressive therapy or for whom immunosuppressive therapy is not indicated.

### Examples

The present invention will be described specifically by the following Examples, but these Examples are merely illustrative examples of the present invention and do not limit the scope of the present invention. In the following Examples, Romiplate (R) was used as a Romiplostim preparation, and this is one example of the pharmaceutical composition comprising Romiplostim as an active ingredient.

### [Example 1]

### Phase II clinical (Ph2) trial

A randomized open-label parallel-group comparative dose-finding study (Ph2 trial) was conducted according to the following protocol, targeting at subjects with aplastic anemia who are refractory to immunosuppressive therapy (hereinafter also referred to as subjects).

### Administration period

### - Fixed dose period (initial dose evaluation period): Weeks 1 to 8

Romiplostim was administered at a dose of either 1 µg/kg, 3 µg/kg, 6 µg/kg or 10 µg/kg once a week for 8 weeks.
The initial dose was maintained during this period, and no dose adjustment was performed, except when satisfying the criteria for discontinuation in 3) below.

### - Extension period: Weeks 9 to 52

The initial dose for the extension period was decided for each patient based on the efficacy and safety data of the fixed dose period (initial dose evaluation period). If a subject does not show a platelet response at week 9, the dose was increased by one level according to the dose adjustment table (extension period). If no platelet measurement is available due to the platelet transfusion, then that week's platelet response was considered as having no response.

### Method for adjusting dose and administration (Weeks 9 to 52)

### 1) Dose escalation

During the extension period, dose escalation was allowed by one level according to the dose adjustment table (extension period). After the dose was increased, if a subject does not show a platelet response even after 4 weeks of administration of Romiplostim, it was allowed to increase the dose by one level, with the highest dose being 20 µg/kg. The necessity and timing of dose escalation after administering the same dose continuously for 4 weeks was decided by a doctor based on the safety and drug efficacy data for each subject.

### 2) Dose adjustment

If a subject showed a platelet response, the dose was increased or decreased by one level according to the "Dose adjustment table (extension period)" (Table 2) and adjusted to an appropriate dose, at the discretion of a doctor to maintain the platelet response while referring to the efficacy and safety data of each subject. The highest dose was set to 20 µg/kg.

**[Table 2]**

| Dose adjustment table (extension period) |
|---|
| **Romiplostim Dose** |
| 1 µg/kg |
| 3 µg/kg |
| 6 µg/kg |
| 10 µg/kg |
| 13 µg/kg |
| 16 µg/kg |
| 20 µg/kg |

### 3) Criteria for discontinuation and resuming

If the platelet count exceeded 400 × 10⁹/L, the treatment was temporarily discontinued. When the platelet count fell to below 200 × 10⁹/L, administration was resumed at a reduced dose by one level from the dose before discontinuation, according to the dose adjustment table (Table 2). Once the platelet count fell to 50 × 10⁹/L or less at that dose, the dosing was changed to the temporarily discontinued dose.

If the platelet count exceeded 200 × 10⁹/L, the dose was decreased by one level, according to the dose adjustment table (Table 2). If the temporarily discontinued dose was 1 µg/kg, the dose when resuming administration was set to 1 µg/kg.

Other than the reasons above, the doctor can reduce the dose at any time if safety concerns arose.

### Criteria for tri-lineage response

The primary endpoint was set to the proportion of subjects achieving a platelet response at week 9, and the main secondary endpoints were set to the proportion of subjects achieving a platelet response, the time to platelet response, the proportion of subjects who became platelet transfusion independent, and the proportion of subjects achieving erythroid response and/or neutrophil response. These evaluation indicators were defined as follows.

Achieving tri-lineage response or tri-lineage response positive is defined as achieving platelet response, erythroid response and neutrophil response all together.

### Definition of evaluation indicators

◆ Platelet response: when any of the following applies
   - the platelet count has increased by 20 × 10⁹/L or more above baseline (the level before start of administration of Romiplostim)
   - the platelet count is 10 × 10⁹/L or more, and has increased by 100% or more from baseline

   However, the evaluation result for subjects within 7 days after platelet transfusion was set as "no response".
◆ Erythroid response: when any of the following applies
   - the hemoglobin concentration has increased by 1.5 g/dL or more above baseline without red blood cell transfusion in subjects with a hemoglobin concentration of less than 9.0 g/dL before Romiplostim administration
   - the transfusion unit has reduced by 4 units or more for 8 consecutive weeks compared with the red blood cell transfusion requirement in the 8 weeks prior to Romiplostim administration

   However, the evaluation result for subjects within 28 days after red blood cell transfusion was set as "no response".
◆ Neutrophil response: when any of the following applies
   - the neutrophil count has increased by 100% or more over the baseline in subjects with a neutrophil count of less than 0.5 × 10⁹/L before Romiplostim administration
   - the neutrophil count has increased by 0.5 × 10⁹/L or more over the baseline in subjects with a neutrophil count of less than 1.0 × 10⁹/L before Romiplostim administration

However, the evaluation result for subjects within 7 days after G-CSF administration was set as "no response".

### ◆ Transfusion Independence

Platelet transfusion or red blood cell transfusion free period of at least 8 consecutive weeks is achieved in subjects who received platelet transfusion or red blood cell transfusion as pretreatment 8 weeks prior to Romiplostim administration.

### Results

The results of the Ph2 trial are shown in Table 4 and Figures 1 to 3 together with the results of the Ph2/3 trial of Example 2.

Subcutaneous administration was started once a week at any dose of 1, 3, 6, or 10 µg/kg, and no dose adjustment (increase or decrease) was observed during the fixed dose period (initial dose evaluation period) until week 8. The proportion of subjects showing a platelet response at week 9, which was the primary endpoint of efficacy, increased with the dose escalation, and was 0% in the 1 and 3 µg/kg groups, 33.3% in the 6 µg/kg group and 70.0% in the 10 µg/kg group. In addition, the proportion of subjects showing an erythroid response and/or neutrophil response during the initial dose evaluation period also increased with dose escalation, and was 14.3% in the 1 µg/kg group, 57.1% in the 3 µg/kg group, 55.6% in the 6 µg/kg group and 70.0% in the 10 µg/kg group. From the above results, 10 µg/kg was found to achieve a significant platelet response, erythroid response and/or neutrophil response during the initial dose evaluation period and was set as the treatment starting dose of the Phase II/III clinical (Ph2/3) trial described later.

### [Example 2]

### Phase II/III clinical (Ph2/3) trial

An international joint open-label intra-individual dose adjustment phase II/III clinical trial was conducted according to the following protocol, targeting at subjects with aplastic anemia who were refractory to immunosuppressive therapy or for whom immunosuppressive therapy is not indicated.

### Administration period (Weeks 1 to 52)

Romiplostim is administered subcutaneously once a week according to the following.

### - Fixed dose period (Weeks 1 to 4)

The initial dose is set to 10 µg/kg, and the dose was fixed between weeks 1 and 4.

### - Extension period (Weeks 5 to 52)

From week 5, the dose may be adjusted according to the following dose adjustment.

### Dose adjustment

### 1) Dose escalation

If a subject does not show a platelet response for 4 consecutive weeks, the dose was increased by one level according to the dose adjustment table (Table 3). However, even if a subject does not show a platelet response, if there were concerns about the expression of adverse events, deterioration, or the like, the dose of the study drug may be maintained at the discretion of a doctor.

In addition, even if a subject shows a platelet response, if deemed necessary by the doctor, it was allowed to increase the dose by one level every 4 weeks.

**[Table 3]**

| Dose adjustment table |
|---|
| **Romiplostim Dose** |
| 5 µg/kg |
| 10 µg/kg |
| 15 µg/kg |
| 20 µg/kg |

### 2) Dose decrease and discontinuation

At a dose of 10 to 20 µg/kg, if the same dose was administered for 8 consecutive weeks or more, and during this period, all the following tri-lineage values were maintained for 8 weeks without transfusion, the dose was decreased by one level according to the dose adjustment table (Table 3). Furthermore, if the decreased dose was administered for 4 consecutive weeks, and during this period, all the following tri-lineage values were maintained without transfusion, the dose was further decreased by one level according to the dose adjustment table (Table 3).

In addition, at a dose of 5 µg/kg, if the same dose was administered for 4 consecutive weeks or more, and during this period, all the following tri-lineage values were satisfied for 4 weeks without transfusion, Romiplostim was discontinued.

However, during the extended administration period, if the doctor considered that the following tri-lineage values could not be maintained due to dose decrease or discontinuation, it was allowed to postpone dose decrease or discontinuation.
- Platelet count: >50 × 10⁹/L
- Hemoglobin concentration: >10.0 g/dL
- Neutrophil count: >1 × 10⁹/L

### 3) Criteria for dose re-escalation and resuming administration

While tapering, if either of the platelet count, hemoglobin concentration or neutrophil count dropped to the following values, tapering was stopped and the dose was increased by one level according to the dose adjustment table (Table 3). While discontinuation, if either of the platelet count, hemoglobin concentration or neutrophil count dropped to the following values, administration was resumed at 5 µg/kg.
- Platelet count: <30 × 10⁹/L
- Hemoglobin concentration: <9.0 g/dL
- Neutrophil count: <0.5 × 10⁹/L

### 4) Criteria for dose maintenance

While tapering, if a subject does not meet the criteria for tapering or re-escalation the dose, the dose was kept stable and administration was continued.

### Criteria for tri-lineage response

◆ Platelet response: when any of the following is satisfied
   - the platelet count has increased by 20 × 10⁹/L or more above the baseline
   - the platelet count is 10 × 10⁹/L or more, and has increased by 100% or more from the baseline
   - no platelet transfusion has been performed for 8 consecutive weeks in subjects who received platelet transfusion as pretreatment in the 8 weeks prior to the first administration
◆ Erythroid response: when any of the following is satisfied
   - the hemoglobin concentration has increased by 1.5 g/dL or more without red blood cell transfusion in subjects with a baseline hemoglobin concentration of less than 9.0 g/dL
   - the volume of transfusion has cumulatively decreased by 800 mL or more for 8 consecutive weeks in subjects who received red blood cell transfusion as pretreatment in the 8 weeks prior to the first administration
◆ Neutrophil response: when any of the following is satisfied
   - in subjects with a baseline neutrophil count of less than 0.5 × 10⁹/L, the neutrophil count has increased by 100% or more from the baseline
   - in subjects with a baseline neutrophil count of less than 1 × 10⁹/L, the neutrophil count has increased by 0.5 × 10⁹/L or more over the baseline

Achieving tri-lineage response or tri-lineage response positive is defined as achieving platelet response, erythroid response and neutrophil response all together.

### ◆ Transfusion Independence

Platelet transfusion or red blood cell transfusion free period of at least 8 consecutive weeks is achieved in subjects who received platelet transfusion or red blood cell transfusion as pretreatment in 8 weeks prior to Romiplostim administration.

### Results

The results of the Ph2/3 trial are shown in Table 4 and Figures 1 to 3 together with the results of the Ph2 trial of Example 1.

**[Table 4]**

| Proportion of subjects showing tri-lineage response in Ph2 (10 µg/kg group) and all cases in Ph2/3 | | |
|---|---|---|
| | Week 27 | Week 53 |
| Ph2 (10µg/kg group) | 0% (0/10 subjects) | 10.0% (1/10 subjects) |
| Ph2/3 | 25.8% (8/31 subjects) | 38.7% (12/31 subjects) |

The proportion of subjects showing hematological response (either platelet response, erythroid response or neutrophil response) at week 27 was 83.9% in total. From the results of the present study, it became clear that a significant hematologic response (platelet response, erythroid response and/or neutrophil response) could be obtained by starting subcutaneous weekly administration of Romiplostim at 10 µg/kg to AA patients who are refractory to immunosuppressive therapy including ATG or for whom immunosuppressive therapy is not indicated, and then adjusting the dose of Romiplostim as appropriate to 5 to 20 µg/kg, with platelet response and platelet count, and adverse events as indicators.

### Discussion

In the Ph2/3 trial, the dose increment is larger (5 µg/kg vs. 3 to 4 µg/kg) and the initial fixed dose period is shorter (4 weeks vs. 8 weeks) compared to the Ph2 trial. Moreover, the period up to the maximum dose of 20 µg/kg is also short (at least 9 weeks vs. 17 weeks).

The proportion of patients with a tri-lineage response positive was higher in the Ph2/3 trial (31 subjects in total) than in 10 subjects of the group starting with 10 µg/kg initial dose in the Ph2 trial (hereinafter also referred to as Ph2 (10 µg/kg group)) both at week 27 and week 53. The proportion was further increased from week 27 to week 53. It is considered that the difference in drug efficacy between Ph2 and Ph2/3 is based on the differences in the duration of the fixed dose period and the dose adjustment method. It was found that a strong drug efficacy is exhibited compared to known administration methods, and that the drug efficacy is maintained even after one year, by increasing the initial dose of 10 µg/kg to 20 µg/kg in a short time period according to the administration method of the Ph2/3 trial. In addition, even from week 17, when the dose could be increased to 20 µg/kg in the Ph2 (10 µg/kg group), the difference in drug efficacy with the Ph2/3 group was observed for a long time. When considering the transition of platelet, hemoglobin level and neutrophil level in Figure 1 to Figure 3, the Ph2/3 group was higher than the Ph2 (10 µg/kg group) continuously until week 53 for any of the platelet count (Figure 1), hemoglobin level (Figure 2) and neutrophil count (Figure 3).

It is possible to reach the maximum dose early on by setting the fixed dose period (10 µg/kg) to 4 weeks, then adjusting the dose by a dose increment of 5 µg/kg/week according to the dose and administration of the Ph2/3 trial. According to this dose and administration, high drug efficacy (increased platelet, hemoglobin level and/or neutrophil level) can be achieved, and the effect continues even after reaching the maximum dose. As a result, an excellent improvement effect can be expected even in AA patients who are refractory to immunosuppressive therapy or for whom immunosuppressive therapy is not indicated.

### Industrial Applicability

According to the present invention, high drug efficacy can be achieved in the treatment of aplastic anemia with Romiplostim; and a treatment effective for aplastic anemia patients for whom sufficient effect was not obtained with conventional treatment such as immunosuppressive therapy can be provided.

### Sequence Listing Free Text

SEQ ID No.1: Thrombopoietin fusion protein analog

## Claims

1. Romiplostim for use in the treatment of aplastic anemia, wherein Romiplostim is administered subcutaneously once a week, and
wherein Romiplostim is administered at 10 µg/kg/week for 4 weeks from the start of administration, and administered at more than 10 µg/kg/week and a maximum of 20 µg/kg/week from week 5, wherein:
(A)
(i) Romiplostim is administered at 15 µg/kg/week from week 5 to week 8 and administered at a maximum of 20 µg/kg/week from week 9, and wherein the same dose is maintained for 4 weeks or more after a dose change, or
(ii) Romiplostim is administered at 15 or 20 µg/kg/week from week 5, and wherein the same dose is maintained for 4 weeks or more after a dose change; and/or
(B) a dose increment of Romiplostim from week 5 is 5 µg/kg.

2. Romiplostim for use according to claim 1(B), wherein the same dose is maintained for 4 weeks or more after a dose change.

3. Romiplostim for use in the treatment of aplastic anemia, wherein Romiplostim is administered subcutaneously once a week, and
wherein Romiplostim is administered at 10 µg/kg/week for 4 weeks from the start of administration, and a dose increment of Romiplostim from week 5 is 5 µg/kg.

4. Romiplostim for use according to claim 3, wherein the same dose is maintained for 4 weeks or more after a dose change.

## Patentansprüche

1. Romiplostim zur Verwendung in der Behandlung von aplastischer Anämie, wobei Romiplostim einmal in der Woche subkutan verabreicht wird, und
wobei Romiplostim zu 10 µg/kg/Woche für 4 Wochen ab dem Anfang der Verabreichung verabreicht wird, und zu mehr als 10 µg/kg/Woche und ein Maximum von 20 µg/kg/Woche ab Woche 5 verabreicht wird, wobei
(A)
(i) Romiplostim zu 15 µg/kg/Woche ab Woche 5 bis Woche 8 verabreicht wird und ein Maximum von 20 µg/kg/Woche ab Woche 9 verabreicht wird, und wobei die gleiche Dosis für 4 Wochen oder mehr nach einer Dosisänderung beibehalten wird, oder
(ii) Romiplostim zu 15 oder 20 µg/kg/Woche ab Woche 5 verabreicht wird, und wobei die gleiche Dosis für 4 Wochen oder mehr nach einer Dosisänderung beibehalten wird; und/oder
(B) eine Dosissteigerung von Romiplostim ab Woche 5 5 ug/kg beträgt.

2. Romiplostim zur Verwendung gemäß Anspruch 1(B), wobei die gleiche Dosis für 4 Wochen oder mehr nach einer Dosisänderung beibehalten wird.

3. Romiplostim zur Verwendung in der Behandlung von aplastischer Anämie, wobei Romiplostim einmal in der Woche subkutan verabreicht wird, und
wobei Romiplostim zu 10 µg/kg/Woche für 4 Wochen ab dem Anfang der Verabreichung verabreicht wird, und eine Dosissteigerung von Romiplostim ab Woche 5 5 ug/kg beträgt.

4. Romiplostim zur Verwendung gemäß Anspruch 3, wobei die gleiche Dosis für 4 Wochen oder mehr nach einer Dosisänderung beibehalten wird.

## Revendications

1. Romiplostim à utiliser dans le traitement de l'anémie aplasique, dans lequel le romiplostim est administré par voie sous-cutanée une fois par semaine, et
dans lequel le romiplostim est administré à raison de 10 µg/kg/semaine pendant 4 semaines à compter du début de l'administration, et est administré à raison de plus de 10 µg/kg/semaine et d'un maximum de 20 µg/kg/semaine à partir de la semaine 5, dans lequel :
(A)
(i) le romiplostim est administré à raison de 15 µg/kg/semaine à partir de la semaine 5 jusqu'à la semaine 8 et est administré à raison au maximum de 20 µg/kg/semaine à partir de la semaine 9, et dans lequel la même dose est maintenue pendant 4 semaines ou plus après un changement de dose, ou
(ii) le romiplostim est administré à raison de 15 ou 20 µg/kg/semaine à partir de la semaine 5, et dans lequel la même dose est maintenue pendant 4 semaines ou plus après un changement de dose ; et/ou
(B) une augmentation de la dose de romiplostim à partir de la semaine 5 est de 5 µg/kg.

2. Romiplostim à utiliser selon la revendication 1(B), dans lequel la même dose est maintenue pendant 4 semaines ou plus après un changement de dose.

3. Romiplostim à utiliser dans le traitement de l'anémie aplasique, dans lequel le romiplostim est administré par voie sous-cutanée une fois par semaine, et
dans lequel le romiplostim est administré à raison de 10 µg/kg/semaine pendant 4 semaines à compter du début de l'administration, et une augmentation de la dose de romiplostim à partir de la semaine 5 est de 5 µg/kg.

4. Romiplostim à utiliser selon la revendication 3, dans lequel la même dose est maintenue pendant 4 semaines ou plus après un changement de dose.
